# EUROPÄISCHE PATENTSCHRIFT

(11) **EP 1 173 272 B1**
(45) Veröffentlichungstag und Bekanntmachung des Hinweises auf die Patenterteilung: **05.02.2003**
(21) Anmeldenummer: 00922639.0
(22) Anmeldetag: 14.04.2000
(51) Int. Cl.: B01F 9/00, A61F 2/06, C12M 3/04

(54) **GEWEBEKULTURSYSTEM ZUR EPITHELIALISIERUNG BZW. ENDOTHELIALISIERUNG UND ZUR FUNKTIONELLEN UNTERSUCHUNG UND ANLIEFERUNG NATÜRLICHER ODER KÜNSTLICHER HOHLORGANE BZW. GEFÄSSE UNTER KONTROLLIERBAREN STERILBEDINGUNGEN FÜR CHIRURGISCHE IMPLANTATIONSZWECKE**
TISSUE CULTURE SYSTEM FOR THE EPITHELIALIZATION OR ENTOTHELIALIZATION AND FOR FUNCTIONALLY TESTING AND SUPPLYING NATURAL OR ARTIFICIAL HOLLOW ORGANS OR VESSELS UNDER CONTROLLED STERILE CONDITIONS FOR THE PURPOSE OF SURGICAL IMPLANTATIONS
SYSTEME DE CULTURE CELLULAIRE PERMETTANT L'EPITHELIALISATION OU L'ENDOTHELIALISATION AINSI QU'UNE SURVEILLANCE DE LA FONCTION D'ORGANES CREUX NATURELS OU ARTIFICIELS NOTAMMENT DE VAISSEAUX ET L'APPROVISIONNEMENT DE CEUX-CI DANS DES CONDITIONS DE STERILITE CONTROLEE EN VUE D'UNE IMPLANTATION CHIRURGICALE

(30) Priorität: 23.04.1999 DE 19918558; 13.08.1999 DE 19938518
(43) Veröffentlichungstag der Anmeldung: 23.01.2002
(73) Patentinhaber: Vascular Biotech GmbH, 80331 München (DE)
(72) Erfinder: NEES, Stephan, D-81375 München (DE); LAMM, Peter, D-82256 Fürstenfeldbruck (DE); JUCHEM, Gerd, D-81379 München (DE)
(74) Vertreter: von Puttkamer, Nikolaus, Dipl.-Ing.
(86) Internationale Anmeldenummer: EP0003420
(87) Internationale Veröffentlichungsnummer: WO00064569

(56) Entgegenhaltungen:
- EP-A- 0 320 441
- WO-A-93/01843
- WO-A-97/49799
- WO-A-98/32367
- US-A- 5 035 708
- US-A- 5 155 035
- US-A- 5 492 826

## Beschreibung

Die vorliegende Erfindung betrifft ein Gewebekultursystem zur Epithelialisierung bzw. Endothelialisierung und zur funktionellen Untersuchung und Anlieferung natürlicher oder künstlicher Hohlorgane bzw. Gefäße unter kontrollierbaren Sterilbedingungen für chirurgische Implantationszwecke nach dem Oberbegriff des Patentanspruches 1.

Insbesondere betrifft die vorliegende Erfindung ein System zur sterilen Aussaat, Kultivation, Funktionskontrolle und Anlieferung von Endothelzellen in natürlichen oder künstlichen Gefäßzylindern- insbesondere in kryopräservierten und vorher deendothelialisierten Blutgefäßen- sowie deren Anlieferung zum Zwecke der chirurgischen Transplantation bzw. Implantation.

### Hintergrund der Erfindung

Der Einsatz eines funktionsfähigen Blutgefäßersatzes spielt in allen chirurgischen Therapiebereichen eine immer größere Rolle. Besonders häufig wird der arterielle Gefäßersatz im kardiochirurgischen Bereich praktiziert. Beispielsweise erfolgt dies bei den sogenannten "Bypass-Operationen". Wenn es irgendwie möglich ist, wird im Primärfall versucht, die Patienten unter Verwendung von autologem Gefäßmaterial, also zum Beispiel durch herauspräparierte Segmente von Gefäßen (Vena saphena magna, Vena saphena parva oder Arteria mammaria interna) zu behandeln, deren Funktion im Entnahmebereich weitgehend von kommunizierenden, verbleibenden Gefäßen übernommen werden kann. Die Situation entwickelt sich allerdings im Einzelfall dann schwierig, wenn solche Gefäße auf Grund einer ungenügenden Qualität (z. B. bei Varikosis oder Arteriosklerose) nicht verwendet werden können. Auf jeden Fall ergibt sich ein Engpaß in der chirurgischen Gefäßversorgung aber bei der zunehmenden Zahl aortokoronarer Reoperationen vor dem Hintergrund des früher bei demselben Patienten schon einmal stattgefundenen Verbrauchs von autologem Gefäßmaterial. Die Gewinnung weiterer körpereigener Venen oder Arterien, wie zum Beispiel der Arteria gastroepiploica oder der Arteria radialis ist für den Patienten sehr aufwendig und risikovoll, und kommt daher nur selten in Frage.

Aus diesem Grunde gibt es deshalb meist nur die Alternative des prosthetischen Gefäßersatzes. Hierfür standen im Prinzip zunächst nur flexible Röhren aus biologisch inertem Kunststoff (wie z. B. Teflon) zur Verfügung, deren Einsatz vor allem am Herzen auf Grund einer hohen Oberflächenthromgenität allerdings nur wenig erfolgreich war. Bereits vor 20 Jahren wurden daher Versuche unternommen, durch kryopräservierte (d. h. in flüssigem Stickstoff bis zum Bedarfsfall eingefrorene) Spendervenen die Lücke bei dem erforderlichen Bypassmaterial zu schließen. Die Ergebnisse nach der Implantation sind jedoch ernüchternd, weil immer noch viele der bisher eingesetzten Gefäßprothesen auch dieser Art schon bald im Patienten thrombosierten. Dennoch sind diese "kryopräservierten Allografts" nach dem fehlgeschlagenen Versuch eines autologen Gefäßersatzes notgedrungenermaßen bis heute das geeignete Mittel der Wahl für aortokoronare Bypass-Reoperationen bzw. bei primären Bypass-Patienten ohne ausreichendes autologes Gefäßmaterial.

Ein vielversprechender Ausweg bietet sich dadurch an, daß man chirurgische Gefäßprothesen vor ihrer Implantation mit vaskulärem Endothel auskleidet, das zur Vermeidung immunologischer Abstoßungsreaktionen allerdings vom Patienten selbst stammen muß, sogenanntes 'autologes Endothel' (Lamm P., Juchem G., Weyrich P., Nees S., Reichart B.: New alternative coronary bypass graft: First clinical experience with an autologous endothelialized cryopreserved allograft. J. Thorac. Cardiovasc. Surg. (1999), 117 (6): 1217-1219). Vaskuläres Endothel begründet durch aktive Entfaltung zahlreicher antiaggregatorischer, antikoagulatorischer und profibrinolytischer Aktivitäten die schon lange bekannte, heute aber auch detailliert erklärbare Antithrombogenität aller gesunden Gefäßwände im Herz- und Kreislaufsystem.

Die enzymatische Isolierung vaskulärer Endothelzellen auch aus menschlichen Blutgefäßen ist heute standardmäßig durchführbar. Eine gleichmäßige Aussaat dieser Zellen in fibrinbeschichteten PTFE - Prothesen wurde durch Rotation eines mit der Zellsuspersion gefüllten Gefäßes um seine longitudinale Achse erreicht. Nach etwa 3 Stunden erfolgt die Entnahme der mit den adhärent gewordenen Endothelzellen besiedelten Gefäße und ihre Überführung in spezielle Kultivationsgefäße. Das Auswachsen der Endothelzellen bis zur kontinuierlichen Auskleidung der Gefäße ("Kultivation bis zur Konfluenz") wird in herkömmlichen Brutschränken bei 37°C in gesättigter Wasserdampfatmosphäre erreicht.

Dieses Verfahren wurde auch schon erfolgreich zur autologen Endotheliasierung kryopräservierter und zuerst von ihrem nativen Endothel befreiter Venen eingesetzt, wobei die Kultivationsphase unter ständiger Überdruckperfusion der mit Endothelzellen besiedelten Venen verlief (s. oben zitierte Publikation).

Bei allen bislang bekannten Verfahren besteht das prinzipielle Problem, daß die zu endothelialisierenden Blutgefäße zum Zwecke ihrer Vorbereitung, zur Aussaat der Endothelzellen für den Kultivationsprozeß und für den Qualitätsnachweis des schließlich erhaltenen Gefäßpräparates mehrfach umgelagert und neu montiert werden müssen. Abgesehen von der Gefahr einer erheblichen mechanischen Schädigung der empfindlichen Prothesen, zum Beispiel durch Kollabieren und durch Reibungseffekte vor allem im Bereich der Innenwand, steht die unter diesen Bedingungen unvermeidlich hohe mikrobielle Kontaminationsgefahr einer verantwortlichen klinischen Nutzung solcher Präparate entgegen. Aus analogen Gesichtspunkten ist auch die Kultivation der Gefäße für den medizinischen Einsatz in herkömmlichen Brutschränken problematisch, ganz abgesehen davon, daß bei einer Serienproduktion endothelialisierter Gefäße wegen des großen Platz- und ständigen Umrüstungsbedarfes sehr viele Brutschränke, sterile Arbeitsbänke und große Zellkulturräume nötig wären. Weiterhin ist eine akzeptierbare Qualitätskontrolle der endothelialisierten Gefäße nur möglich, wenn das Produkt unmittelbar vor seiner Implantation in den Patienten in möglichst derselben geometrischen Ausrichtung überprüft wird, in der es schließlich auch eingenäht wird. Es kommt hinzu, daß die bisherigen logistischen Maßnahmen für diesen Gefäßersatz auch keinerlei Sorge dafür tragen, wie die endothelialisierte Prothese über teilweise weite Strecken am sichersten und schonendsten in den Operationsraum zu befördern ist.

Das Dokument WO-A-5301843 beschreibt ein Gewebekultursystem, das ein mit Endkappen, Klemmdichtungen und Kanülen verschenes Rohr aufweist.

Die Aufgabe der vorliegenden Erfindung besteht daher darin, ein Gewebekultursystem für eine praktikable Endothelialisierung bzw. Epithelialisierung von Hohlorganen, insbesondere von Blutgefäßprothesen zu schaffen, die eine praktikable und qualitativ akzeptable Beschichtung von Gefäßen mit Endothel- bzw. Epithelzellen ermöglicht und für den Betrieb in handelsüblichen Sterilbänken und Isolatoren geeignet ist, um die aufwendige und teure Inbetriebnahme von Reinst-Räumen umgehen zu können. Außerdem sollte das Problem der sterilen Transportierbarkeit der fertigen endothelialisierten Prothesenprodukte bis in den Operationsraum und das der dortigen sterilen Entnahme gelöst werden.

Diese Aufgabe wird durch die Merkmale des Patentanspruches 1 und 24 gelöst.

Die erfindungsgemäße Einrichtung sieht konzentrische Beschichtungskompartimente bzw. Beschichtungskammern vor, in deren zentralem Innenbereich biologisch zu beschichtende, natürliche oder nicht natürliche Hohlorgane gelagert und fixiert werden können. Bevorzugte Hohlorgane weisen an ihrer inneren Oberfläche bzw. an der luminalen Oberfläche keine Auskleidung mehr mit Epithel oder Endothel des Spenders auf. Eine besonders gestaltete Ausführungsform der vorliegenden Erfindung zielt auf solche Blutgefäße und ihre Klappen ab, die dann mit patientenautologem Endothel ausgekleidet werden.

Diese Beschichtungseinrichtung (System) mit den betriebsbereiten Beschichtungskammern besteht aus einem nicht toxischen Werkstoffen, die in übersättigtem Wasserdampf ausreichende Zeiten lang bei 127° C autoklaviert werden können. Auf diese Weise läßt sich eine vollkommene Sterilisation erreichen.

Ein weiterer Vorteil der vorliegenden Erfindung besteht darin, daß diese Beschichtungseinrichtung mit den Beschichtungskammern fest auf einer speziellen Rotationseinrichtung gelagert und durch diese in Drehung versetzt werden können.

Die sterilen Beschichtungskammern der erfindungsgemäßen Beschichtungseinrichtung lassen über entsprechende Kanülen, Schläuche und 3-Wege-Ventile einen ständigen Zugang in den Innen- und Außenraum der Hohlorgane zu. Durch einfaches Umschalten der Zugangswege zu den entsprechenden Versorgungsgefäßen lassen sich im Zusammenhang mit der wahlweise einschaltbaren Rotationseinrichtung sämtliche Vorarbeiten, Aussaatmaßnahmen, Kultivationszwecke und die Durchführung exakter und zuverlässiger Qualitätskontrollen vornehmen, ohne daß das zentral gelagerte Hohlorgan zwischenzeitlich aus der Beschichtungseinrichtung herausgenommen werden muß. Dadurch ergibt sich vorteilhafterweise ein erheblich geringeres Kontaminationsrisiko. Die qualitative Sorgfaltspflicht wird erfüllt.

Darüber hinaus wird das Kontaminationsrisiko bei einer bevorzugten Ausgestaltung der Erfindung auch dadurch erheblich verringert, daß die notwendigerweise bei 37° C stattfindende Aussaat und Kultivation der Epithel- bzw. Endothelzellen nicht in herkömmlichen Brutschränken stattfindet, sondern unter Temperierung durch einen eigenen Flüssigkeitswärmemantel in einer Temperiereinrichtung erfolgen kann. Als Temperierflüssigkeiten werden erfindungsgemäß desinfizierende Lösungen ausgewählt, die in einem geschlossenen Temperierkreisauf umgewälzt werden.

Ein weiterer wesentlicher Vorteil dieser Ausgestaltung der vorliegenden Erfindung besteht darin, daß zur Temperierung der Beschichtungskammern nur ein verhältnismäßig geringer technischer und räumlicher Aufwand erforderlich ist. Dadurch ergibt sich die Möglichkeit, sehr viele Beschichtungskammern gleichzeitig oder auch zeitlich versetzt auf engstem Raum, vorzugsweise in einer sterilen Arbeitsbank, in normaler Luftatmosphäre nebeneinander zu betreiben.

Die erfindungsgemäße Konstruktion der Beschichtungskammer umfaßt auch die Möglichkeit eines konstanten Atemgasaustausches der wachsenden Zellen unter Konstanthaltung des pH-Wertes im Wachstumsmedium ohne größeren apparativen Aufwand.

Durch die erfindungsgemäße Beschichtungseinrichtung wird es vorteilhafter Weise ermöglicht, daß das in der zentralen Beschichtungskammer eingekapselte Hohlorgan unter Wahrung strikt aseptischer Kautelen unproblematisch aus der vorliegenden Beschichtungseinrichtung entnommen werden kann. Außerdem wird vorher auch der sterile Transport über weite Strecken bis zum Operationsraum ermöglicht. Dort läßt sich das fertige und zuvor funktionell überprüfte Hohlorgan auf einfache Weise steril entnehmen und anschließend sofort implantieren.

Die Erfindung kann bei allen künstlichen und natürlichen Hohlorganen und deren Bestandteilen vorteilhaft angewendet werden, beispielsweise bei natürlichen Blutgefäßen und ihren Klappen, Lymphgefäßen und ihren Klappen, Harnleitern, Samenleitern, Bronchien, Gallengängen, Darmabschnitten und bei jeglichem prosthetischen Ersatz solcher Hohlorgane.

Vorteilhafte Ausgestaltungen der Erfindung gehen aus den Unteransprüchen hervor.

Im folgenden werden die Erfindungen deren Ausgestaltungen im Zusammenhang mit den Figuren näher erläutert. Das zeigen:
- Fig. 1a: ein erfindungsgemäßes Gewebekultursystem zur Endothelialisierung von Blutgefäßprothesen;
- Fig. 1b: eine besonders erfindungsgerechte Gestaltung der Abkapselung der über Kanülen versorgten Prothesen mittels spezieller Verschlußkappen;
- Fig. 2: eine schematische Darstellung der Integration der erfindungsgemäßen Einrichtung in ein Gesamtsystem;
- Fig. 3: in schematischer Darstellung den Aufbau einer einfachen Rotationsanordnung;
- Fig. 4: eine schematische Darstellung zur Erläuterung der bevorzugten Temperierung der erfindungsgemäßen Einrichtung im geschlossenen Umwälzkreislauf, und
- Fig. 5: eine Weiterbildung der Erfindung.

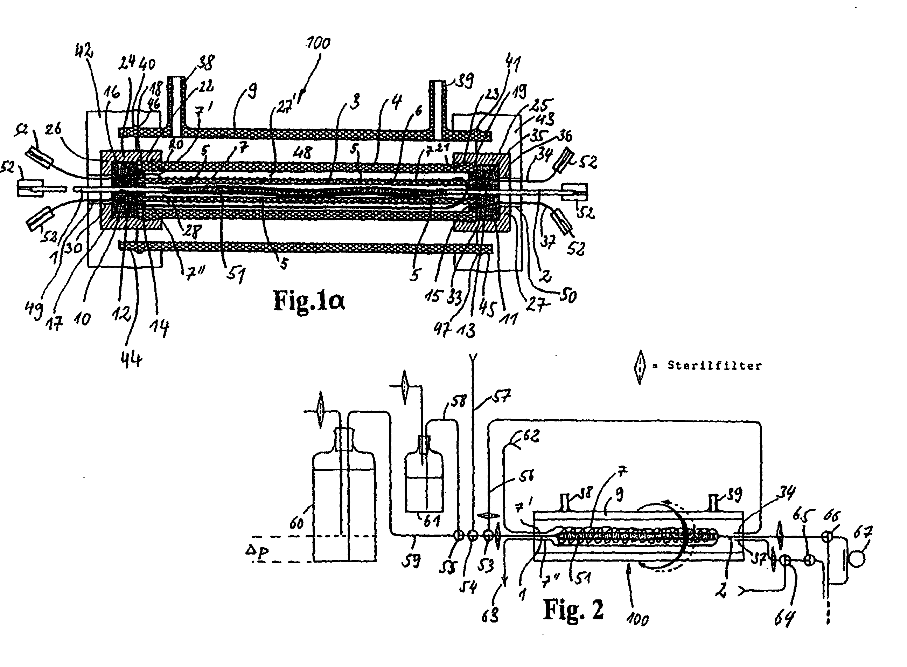

Gemäß Figur 1a umfaßt das vorliegende Gewebekultursystem 100 im wesentlichen eine erste Kanüle 1 und eine zweite Kanüle 2, die vorzugsweise aus Stahl bestehen, ein erstes Rohr 3, ein zweites Rohr 4 und ein Außenrohr 9.

Die Stahlkanülen 1 und 2 verlaufen von außen jeweils in eines der Enden des ersten Rohres 3 hinein und sind in diesem in der Längsrichtung des ersten Rohres 3 von einander beabstandet. Die Kanülen 1 und 2 sind vorzugsweise zweigeteilt, wobei die beiden Teile 1', 1'' jeweils ineinander verschraubbar sind (Fig. 1b).Die zugehörigen Kanülen-Verbindungsstellen werden im Bereich der Quetschdichtung 10 bzw. 11 positioniert und durch diese zirkumferent abgedichtet. Außerdem besitzt auch das aus der Dichtung 10 nach außen ragende Ende der Kanüle 1 bzw. des Teiles 1' ein Schraubgewinde, über das eine Verlängerungskanüle desselben Außendurchmessers angesetzt werden kann, das bei der späteren Einbringung der zu beschichtenden Prothese in das Lumen des Rohres 3 durch das Loch der Quetschdichtung 10 gefädelt wird und dann das Einziehen der an Kanüle 1 fixierten Prothese in das Rohr 3 ermöglicht. Danach wird das Kanülenverlängerungsstück wieder entfernt (siehe hierzu und zu weiteren Details, die noch beschrieben werden Fig. 1b). Vorzugsweise besteht das erste Rohr 3 aus Polycarbonat oder Glas. In dem ersten Rohr 3 sind in einer bewährten Ausführung vier longitudinal verlaufende Reihen mit Löchern angeordnet, die jeweils um 90° gegeneinander in der Umfangsrichtung des ersten Rohres 3 versetzt sind und einen Abstand von ca. 15 mm aufweisen. Die Löcher 5 weisen vorzugsweise jeweils einen Durchmesser von etwa 2 mm auf. In seinem Außenumfang besitzt das erste Rohr 3 eine von der einen Seite des ersten Rohres 3 zur anderen Seite desselben verlaufende schraubenförmige Nut 6, die zur Aufnahme einer dünnwandigen, kleinlumigen Leitung 7 dient, die um das erste Rohr 3 herumgewickelt ist. Die Leitung 7 besteht vorzugsweise aus Teflon. Ihr Durchmesser beträgt etwa 1 - 1,5 mm. Die Wandstärke der Leitung 7 beträgt etwa 0,1 - 0,2 mm.

An jeder Seite des ersten Rohres 3 befindet sich vorzugsweise eine aus Silikon-Kautschuk bestehende Quetschdichtung 10, 11, wobei die Kanüle 1 bzw. 2 durch eine mittlere Bohrung 12 bzw. 13 der Quetschdichtungen 10 bzw. 11 verläuft und der jeweilige Endbereich des ersten Rohres 3 in eine ringförmige Nut 14 bzw. 15 eingesetzt ist, die sich in der dem Rohr 3 zugewandten Seite der jeweiligen Quetschdichtung befindet. Auf diese Weise wird das erste Rohr 3 konzentrisch zu den Kanülen 1 und 2 gehalten. Vorzugsweise bestehen die Quetschdichtungen 10 bzw. 11 aus einem aus 2 Komponenten zusammengesezten, zunächst zähflüssigen Silikon-Kautschuk der Firma Wacker Chemie, München (Elastosil 4601 A bzw. B). Sie werden speziell gegossen und heißpolymerisiert, sind biologisch absolut inert und setzten keinerlei toxische Beimengungen frei. Außerhalb der ringförmigen Nut 14 bzw. 15 weist die in der Figur 1 linke Quetschdichtung 10 Durchgänge 16 bzw. 17 auf, durch die der eine Endbereich 7' der Leitung 7 bzw. der andere Endbereich 7'' der Leitung 7 hindurch geführt sind.

Das erste Rohr 3 ist vorzugsweise konzentrisch in dem zweiten, vorzugsweise aus Glas gefertigten Rohr 4 angeordnet, das mit seinen Enden in eine ringförmige Nut 18 bzw. 19 der Quetschdichtung 10 bzw. 11 eingreift. An seinen Endbereichen weist das zweite Rohr 4 außenseitig jeweils ein Außengewinde 20 bzw. 21 auf, an dem das Innengewinde 22 bzw. 23 eines kappenförmigen, autoklavierbaren Schraubdeckels 24 bzw. 25 verschraubbar ist, wobei die Quetschdichtungen 10 bzw. 11 zwischen der Endwand 26 bzw. 27 des Schraubdeckel 24 bzw. 25 und den jeweiligen Enden des ersten Rohres 3 und des zweiten Rohres 4 zusammen gequetscht werden, wobei die in die Nuten 14 bzw. 15 und 18 bzw. 19 eingreifenden Enden des ersten Rohres 3 und des zweiten Rohres 4 abgedichtet werden, so daß die Beschichtungskammer 28, die den Innenraum des ersten Rohres 3 und den Raum 27' zwischen dem ersten Rohr 3 und dem zweiten Rohr 4 umfaßt, nach außen abgedichtet ist. Ebenso werden beim Zusammendrücken der Quetschdichtungen 10, 11 die Hindurchführungen der Endbereiche 7' und 7'' der Leitung in den Durchgängen 16 und 17 der linken Quetschdichtung 10 abgedichtet. Die Stahlkanüle 1 und die Endbereiche 7', 7'' der Leitung 7 verlaufen durch eine Bohrung 30 in der Endwand 26 des Schraubendeckels 24 nach außen.

Der Innenraum des ersten Rohres 3 bzw. die Beschichtungskammer 28 steht über eine Schlauchleitung 34, die durch einen Durchgang 35 der rechten Quetschdichtung 11 dicht hindurch geführt ist mit der Außenseite in Verbindung. Die Schlauchleitung 34 verläuft durch eine Bohrung 36 der Endwand 27 des rechten Schraubdeckels 25. In einer entsprechenden Weise verläuft eine Schlauchleitung 37 von der Beschichtungskammer 27 durch einen Durchgang 33 der rechten Quetschdichtung 11 und die Bohrung 36 nach außen.

Eine besonders erfindungsgerecht ausgestaltete Konstruktion der beiden Quetschdichtungen 10 und 11 mit ihren Kanülen 1 bzw. 2 ergibt sich aus Fig. 1b, in der allerdings aus Gründen der besseren Übersichtlichkeit die Schlauchleitungen 16 und 17 (der Quetschdichtung 10) bzw. 34 und 37 (der Quetschdichtung 11) weggelassen wurden. Dort sind auch die Mittel skizziert, mit denen es gelingt, die mit den Prothesenendstücken verbundenen Innenteile der zweiteiligen Kanülen 1 und 2 unter sterilen Kautelen von der Außenwelt abzuschirmen, obwohl es andererseits notwendig ist, die Prothesen von außen her zu versorgen, z. B. mit Nährmedium zu perfundieren. Zweckmäßigerweise werden die Quetschdichtungen 10 und 11 auf ihrer Außenoberfläche jeweils mit wenigstens einer das zentrale Kanülenführungsloch konzentrisch umlaufenden Dichtlippe 90 versehen, die mittels der Schraubdeckel 24 und 25 aufpressbare, stempelartige Stahlteile 91 mit Zentralbohrung 92 auf ihrer Auflagefläche zu den Dichtungen 10 bzw. 11 zu ihrer jeweils durchlaufenden Kanüle hin abdichten. Das zylinderartige Ende beider Stahlteile 91 ist mit einem Außengewinde 93 versehen, das das Aufschrauben eines durchlochten, mit einer Silikondichtung 95 versehenen Deckels 94 ermöglicht, mit dessen Hilfe der zu den Dichtungen 10 bzw. 11 führende Teil der Kanülen 1 bzw. 2 zur Außenwelt hin abgeschirmt werden kann. Nach Beendigung der angestrebten Beschichtung der Prothese (siehe unten) können die Außenteile der Kanüle abgeschraubt werden und die im Bereich der Dichtungen 10 und 11 verbleibenden Kanülenstücke 1'', 2'' mit der angehängten Prothese durch Aufschrauben eines Abschlußdeckels 96 mit ungelochter Dichtung 97 am Ende der Stahlteile 91 für den Transport steril verschlossen werden. Es wird darauf hingewiesen, daß in Figur 1b nur die linke Seite mit den Knülenteilen 1' und 1'' dargestellt ist. Die rechte Seite ist entsprechend ausgestaltet.

Konzentrisch zu der bisher beschriebenen Anordnung ist das Außenrohr angeordnet, das vorzugsweise aus einem Glasrohr besteht, das an jedem Endbereich einen Zugangsstutzen 38, 39 aufweist und jeweils einen diesem nach außen vorgelagerten ringförmigen Haltewulst 40 bzw. 41 an der Außenfläche des Außenrohres 9 besitzt. Auf den kappenförmigen Schraubendeckel 24 und den kappenförmigen Schraubendeckel 25, die in der beschrieben Weise das erste Rohr 3 und das zweite Rohr 4 halten, sind jeweils ein Kappenteil 42 bzw. 43 aufgesetzt, wobei die Endbereiche des Außenrohres 9 jeweils in eine ringförmige Nut 44 bzw. 45 des Kappenteiles 42 bzw. 43 eingreifen und die Haltewülste 40 bzw. 41 in entsprechende Vertiefungen 46 bzw. 47 der Kappenteile 42 bzw. 43 eingreifen. Auf diese Weise wird das Außenrohr 9 durch die flexiblen und straff an ihm aufsitzenden Kappenteile 42 und 43 konzentrisch zum zweiten Rohr 4 gehalten, wobei der Raum 48 zwischen dem zweiten Rohr 4 und dem Außenrohr 9 nach außen abgedichtet ist.

Vorzugsweise bestehen auch die Kappenteile 42, 43 aus dem oben bereits bezeichneten, speziell gegossenen und heißpolymerisierten 2-Komponenten Silikon-Kautschuk der Firma Wacker Chemie, München.

Die Kanülen 1, 2 verlaufen durch Bohrungen 49 bzw. 50 des linken bzw. rechten Kappenteiles 42 bzw. 43. Durch die Bohrung 49 des linken Kappenteiles 42 verlaufen auch die Endbereiche 7', 7'' des Schlauches 7 nach außen. Durch die Bohrung 50 des rechten Kappenteiles 43 verlaufen auch die Schlauchleitungen 34, 37 nach außen.

Zu seiner Endothelialisierung wird ein Blutgefäß 51 etwa mittig im ersten Rohr 3 angeordnet, wobei in die Enden des Blutgefäßes 51 die Kanülen 1 bzw. 2 hineinverlaufen. Zur Abdichtung werden die Enden des Blutgefäßes 51 auf den Endbereichen der Kanülen 1, 2 mit einem Faden fixiert.

In der aus der Figur 1 erkennbaren Weise befinden sich an den Enden der Kanülen 1, 2, den Enden der Endbereiche 7', 7'' des Schlauches 7 und den Enden der Schlauchleitungen 34, 37 Kupplungsstücke 52, die vorzugsweise ebenfalls aus dem bereits genannten 2-Komponenten Silikon-Kautschuk bestehen und zur dichten Verbindung mit in der Fig. 1 nicht dargestellten, externen Verbindungsschläuchen dienen.

Auf Grund der Auswahl der oben beschriebenen Materialien kann die gesamte vorliegende Einrichtung im gesättigten Wasserdampf bei einer Temperatur von 121 °C autoklaviert und dadurch absolut zuverlässig auch in allen Hohlräumen sterilisiert werden.

Gemäß Figur 2 wird die vorliegende Gewebekultureinrichtung 100 der Figur 1 mit verschiedenen Versorgungseinheiten verbunden. Einzelheiten der Figur 2, die bereits im Zusammenhang mit der Figur 1 erläutert wurden, sind in der entsprechenden Weise bezeichnet. Im linken Teil der Figur 2 ist dargestellt, daß das Blutgefäß 51 über die Kanüle 1, 3-Wege-Ventile 53, 54, 55 und verschiedene Schlauchleitungen 56, 57, 58, 59 mit verschiedenen Lösungen kontaktiert werden kann: aus einem Gefäß 60 über die Leitung 59 mit Kulturmedium, über die Leitung 58 mit autologem Serum aus einem Gefäß 61, über die Leitung 57 auch noch mit anderen Lösungen bzw. Suspensionen. Die gasdurchlässige Leitung 7 aus dünnwandigem Teflon gestattet eine ausreichende Versorgung der Blutgefäßzellen im sonst vollkommen geschlossenen System mit Sauerstoff und sorgt, wegen der Beimischung von 5 % CO₂ in die durchgeblasene atmosphärische Luft, für eine Konstanz des pH-Wertes des im Inneren der Gewebekultureinrichtung verwendeten Bicarbonat-gepufferten Kulturmediums. Zu diesem Zweck sind der Endbereich 7' der Leitung 7 mit einem Gaseinlaß 62 für atmosphärische Luft und 5% CO₂ und der Endbereich 7'' der Leitung 7 mit einem Gasauslaß 63 verbunden.

Mit der Hilfe der Leitung 7 und bei entsprechender Schaltung der 3-Wege-Ventile 53, 54, 55 und weiterer, in der Figur 2 rechts dargestellter 3-Wege-Ventile 64, 65, kann auch der Außenraum des Blutgefäßes 51 über die Schlauchleitung 56 bzw. 34 mit Kulturmedium versorgt werden, wobei das Kulturmedium nach Durchgang durch die Beschichtungskammer 28 über die Schlauchleitung 37 und die 3-Wege-Ventile 64, 65 zu einem nicht dargestellten Abtropfgefäß gegeben wird. Dies hat zum einen den Vorteil, daß das labile, meist dünnwandige Blutgefäß 51 nicht mit seinem ganzen Gewicht auf dem Unterstützungsrohr aufliegt, sondern im Kulturmedium schwimmt und daß zum anderen bei Applikation eines geringen Überdrucks (ΔP = 10 - 20 cm H₂O) zur Füllung und Perfusion des Blutgefäßes 51 mit Kulturmedium auch eine geringfügige transmurale Filtration dieses Mediums durch die Wandschicht des Blutgefäßes 51 erfolgt, wenn der Weg für diese Filtratmenge über die 3-Wege-Ventile 64, 65 zum Abtropfgefäß freigegeben wird. Dieses Vorgehen fördert den Stoffaustausch der Wandzellen. Bei langfristiger Durchströmung (Perfusion) des Blutgefäßes 51 mit dem - wegen der Zumischung von autologem Patientenserum - wertvollen Kulturmedium empfiehlt sich die Führung des aus der Kanüle 2 abtropfenden Kulturmediums über ein weiteres 3-Wege-Ventil 66 unter der Kontrolle eines periodisch geöffneten Magnetventils 67 oder einer periodisch aktivierten, peristaltischen Schlauchpumpe.

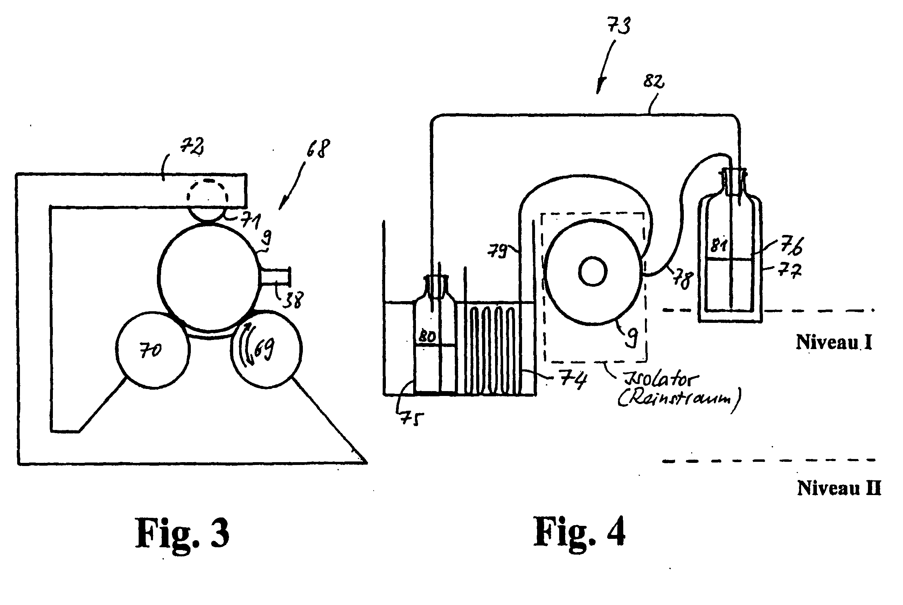

Die Figur 3 zeigt die Seitenansicht des auf einer Rotationseinrichtung 68 positionierten vorliegenden Gewebekultursystems. Eine kontinuierliche und alternierend vorzugsweise mindestens 180° in die eine und dann mindestens 180° in die andere Richtung, 15 - 20 mal pro 1 Stunde rotierende Antriebswalze 69, zweckmäßigerweise mit einem Silikonüberzug, bewirkt eine passive Mitdrehung der Beschichtungseinrichtung. Die ebenfalls passiv mitdrehende Walze 70 dient zusammen mit der Antriebswalze 69 als Unterstützung für das Gewebekultursystem. Ein einfach zu arretierender, mit einem leicht drehbaren Rad 71 versehener Bügel 72 hält die Beschichtungseinrichtung vorzugsweise von oben her sicher zwischen den Walzen 69, 70. Die Längsdimensionierung der Walzen 69, 70 wird vorzugsweise so gewählt, daß sie - bei seitlicher Ansicht - zwischen den beiden Zugangsstutzen 38, 39 des Außenrohres 9 der vorliegenden Beschichtungseinrichtung liegt. Dadurch wird eine Bruchgefahr der Zugangsstutzen 38, 39 ausgeschlossen.

Im Zusammenhang mit der Figur 4 wird nun die bevorzugte Temperierung der vorliegenden Beschichtungseinrichtung im Umwälzprinzip erläutert. Da die Temperierflüssigkeit gleichzeitig auch desinfizierende Eigenschaften haben soll und deswegen möglicherweise auch organische Lösungsmittel (z. B. Äthanol) enthält, erfolgt die Umwälzung zweckmäßigerweise im geschlossenen System. Die Temperiereinrichtung 73 weist zwei über eine zwischengeschaltete Wärmeaustauscherspirale 74 und den Raum 67 zwischen dem Rohr 4 und dem Außenrohr 9 in Serie geschaltete Vorratsflachen 75, 76 auf. Vorratsflasche 75 befindet sich zusammen mit der Wärmeaustauscherspirale 74 im 37°C-warmen Temperierbad bzw. Wasser eines standardmäßigen Umwälzthermostaten. Vorratsflasche 76 wird über einen eigenen Wärmemantel 77 mit dem umgepumpten Temperierwasser auf 37° C vorerwärmt. Die in den Vorratsflaschen und im Wärmemantel der Endothelialisierungseinheit befindlichen Flüssigkeiten kommunizieren über die Schlauchleitungen 78, 79, die Gasräume 80,81 der beiden Flaschen 75, 76 dagegen über den Luftaustauscherschlauch 82.

Wird nun die Vorratsflasche 76 mit der Hilfe einer möglichst einfach gestalteten mechanischen Vorrichtung (nicht dargestellt) alternierend vom Niveau II auf das Niveau I gehoben bzw. wieder auf das Niveau II gesenkt usw. kommt es zu entsprechenden Ausgleichsströmungen von Temperierungsflüssigkeiten in den Leitungen 77, 78 bzw. 81 und damit zur gleichmäßigen Temperierung des Gewebekultursystems bzw. des darin positionierten Blutgefäßes 51. Zweckmäßigerweise wird dieses Temperiersystem seitlich außerhalb der zur Abschirmung der Beschichtungseinheit eingesetzten sterilen Arbeitsbank montiert und nur über die Schläuche 77 und 78 mit deren Innenraum verbunden.

Die beschriebene Beschichtungseinrichtung läßt sich in vorteilhafter Weise auf engstem Raum auch zur Serienbeschichtung mehrerer Blutgefäße gleichzeitig einsetzen, wenn entsprechend viele Gewebekultursysteme auf ihren individuellem oder in Serie geschalteten Rotationseinrichtungen 68 nebeneinander angeordnet und mit Temperierflüssigkeit versorgt werden. Teure Brutschränke entfallen, der Platzbedarf ist relativ gering.

Im folgenden wird die Funktion der erfindungsgemäßen Einrichtung an Hand der autologen Endothelialisierung einer kryopräservierten Vene eines Patienten erläutert.

Die Kryopräservierung, das Auftauen und die Deendotheliasierung der Vene, sowie die Isolierung vom autologem Serum bzw. autologem Endothelzellen und deren Kultivation erfolgt vor der eigentlichen Endothelialisierung aufgrund in der Fachliteratur publizierter Methoden.

Zum besseren Verständnis aller im folgenden beschriebenen Vorgänge wird auf die Figuren 1 bis 4 Bezug genommen.

Die Autoklavierung des Außenrohres 9 der vorliegenden Beschichtungseinrichtung mit den aufgesetzten Kappenteilen 42, 43 erfolgt getrennt von der über die Quetschdichtungen 10, 11 bzw. die Schraubdeckel 24, 25 zusammengehaltenen Rohre 3, 4. In die entsprechend Fig. 1b gestalteten Quetschdichtungen 10, 11 werden zuvor auch die Endbereiche 7', 7'' der Leitung 7 bzw. die Schlauchleitungen 35, 37 eingefädelt und mit ihren Kupplungen 52 versehen.

Nach dem Abkühlen der Teile der vorliegenden Beschichtungseinrichtung wird eine aufgetaute und von ihrem nativen Endothel befreite (deendothelialisierte) Spendervene an ihren Enden mit den zweiteiligen Kanülen 1, 2 (s. Fig. 1b) verbunden. Danach wird das separat autoklavierte Rohrsystem aus den Rohren 3 und 4 vom rechten Ende her durch Aufdrehen des Schraubendeckels 25 mit dem eingelegten Stahlteil (vgl. Fig. 1b) und Entfernen der Quetschdichtung 11 geöffnet. Anschließend wird die an die zweiteilige Kanüle 1 angeschraubte Verlängerungskanüle (vgl. Fig 1b und Text der gerätetechnischen Beschreibung auf Seiten 9 bzw. 11/12) durch die entsprechende Bohrung 12 der gegenüberliegenden Quetschdichtung 10 bzw. die Bohrung 30 des Schraubdeckels 24 geführt. Diese Maßnahme wird durch leichtes Aufdrehen des Schraubendeckels 24 mit eingelegtem Stahlteil erleichtert, damit sich die Quetschdichtung 10 entspannen kann. Auf diese Weise kommt das Blutgefäß 51 schließlich in seiner ganzen Länge ausgestreckt im Inneren des ersten Rohres 3 zu liegen und die rechte, zweiteilige Kanüle 2 kann nun durch die zentrale Bohrung 13 der zuvor abgenommenen, aber schon positionierten Quetschdichtung 11 und die Bohrung 36 des Schraubdeckels 25 eingeführt werden. Nun ist der Verschluß des Doppelrohrsystems mit den Rohren 3 und 4 auch an seinem rechten Ende durch Dichtung 11 und Verschrauben des Schraubdeckels 25 möglich. Auch die beiden Enden der Kanülen 1, 2 erhalten nun Kupplungselemente 52. Schließlich kann die ganze Konstruktion noch mit Hilfe des Außenrohres 9 und der zugehörigen Kappenteile 42, 43 ummantelt werden.

Die fertig montierte Beschichtungseinrichtung wird über die diversen Leitungen 56, 57, 58 und die 3-Wege-Ventile 53, 54, 55, 64, 65, 66 gemäß der Figur 2 verschaltet und gemäß Figur 3 auf einer Rotationseinrichtung 68 positioniert. Die Leitung 7 wird bis zum Ende aller im folgenden geschilderten Schritte von einem Luftgemisch mit 5% Volumenanteil Kohlendioxid durchströmt.

Zur Vorbereitung des Blutgefäßes 51 für die Aussaat der Patienten-autologen Endothelzellen wird es zunächst aus dem Vorratsgefäß 61 über die entsprechend gelegten 3-Wege-Ventile 53, 54 und 55 (Figur 2) zunächst mit Patienten-autologem Serum gefüllt, wobei auch das 3-Wege-Ventil 66 kurzzeitig die verdrängte Luft aus dem Blutgefäß freigeben muß. Es erfolgt eine Inkubation für 12 bis 24 Stunden bei 37° C mit der Hilfe der in der Figur 4 gezeigten Temperiereinrichtung.

Danach wird das Serum durch Eindrücken von steriler Luft über die Leitung 57 und bei geschlossenem 3-Wege-Ventil 55 und geöffneten 3-Wege-Ventilen 54, 53 und 66 ausgedrückt. Sofort danach werden auf analoge Weise durch Kultivation vermehrte und anschließend suspendierte Endothelzellen des Patienten in zuvor durch mikroskopische Auszählung genau bestimmter Konzentration (120.000 pro cm² der Blutgefäß-Innenwand) injiziert. Der Extravasalraum wird über die Schlauchleitung 34 mit Kulturmedium gefüllt, wobei die Luft aus dem in Figur 2 von links nach rechts oben schräg gestellten Gewebekultursystem über das entsprechend gelegte 3-Wege-Ventil 64 und die Schlauchleitung 37 entweicht. Danach wird die in der Figur 3 dargestellte Rotationseinrichtung 68 drei Stunden lang aktiviert (15 bis 20 alternierende Halbdrehungen /h). In diesem Zeitraum kommt es zur gleichmäßigen Anheftung der sedimentierenden Endothelzellen an der Gefäßinnenwand. Die Temperierung bei 37° C bleibt die ganze Zeit und auch während aller folgender Arbeitsschritte bestehen. Nach dem Abschalten der Rotation und dem anschließenden Ausdrücken des Inhaltes des Blutgefäßes 51 (er kann mikroskopisch auf noch suspendierte Endothelzellen überprüft werden!) mit injizierter Luft über die Leitung 57 und das 3-Wege-Ventil 66 wird das Blutgefäß 51 durch entsprechendes Öffnen der 3-Wege-Ventile 53, 54, 55 und 66 mit Kulturmedium gefüllt, das aus dem Gefäß 60 - auf besonders einfache Weise mit Hilfe einer Boyle-Marriott'schen Flasche, aber natürlich möglicherweise auch mit Hilfe elektronischer oder anderer konstanter Druckgeber - unter leichtem Überdruck (ca. 10 - 20 cm H₂O) angeboten wird.

Das aus dem Blutgefäß 51 bzw. dem Venenlumen austretende Kulturmedium wird bei entsprechend geschalteten 3-Wege-Ventil 66 über eine Schlauchstrecke geführt, die nur periodisch (z. B. jede Stunde für ca. 10 Minuten) durch Aktivieren einer peristaltischen Pumpe oder eines Magnetventils 67 freigegeben wird. Auf dies Weise ergibt sich eine besonders sparsame, diskontinuierliche Perfusion des Blutgefäßes 51 mit dem wertvollen Kulturmedium (Medium 199 mit Zusatz von 20 % autologem Serum und rekombinantem basischem Fibroblasten - Wachstumsfaktor in der Konzentration 2 ng/ml). Um eine wegen des leichten hydrostatischen Überdrucks im Blutgefäß 51 mögliche physiologische Durchspülung auch der Gefäßwandschichten durch transmurale Filtration zu erreichen, werden die 3-Wege-Ventile 64 und 65 so geschaltet, daß der Weg vom Extravasalraum in ein steril von der Außenwelt abgeschirmtes Abtropfgefäß freigegeben wird.

Nach 5 bis 7 Tagen diskontinuierlicher Perfusion des Blutgefäßes 51 und der dabei erfolgenden konfluenten Auskleidung seiner Innenfläche mit Patienten-autologem Endothel können im Prinzip diverse Qualitätskontrollen zum Nachweis der Vollständigkeit und Güte der erzielten Endothelbeschichtung durchgeführt werden. Die erforderliche Füllung des Blutefäßes 51 mit speziellen Reaktionslösungen bzw. Substraten wird wieder über die Leitung 57 und durch entsprechende Schaltung der 3-Wege-Ventile 53, 54, 55 bzw. Öffnen des 3-Wege-Ventils 66 erreicht.

Schließlich wird das Blutgefäß 51 noch einmal sorgfältig mit Kulturmedium durchspült und unter leichtem Überdruck gefüllt. Alle Kupplungsstücke 52 werden abgeklammert, dann werden alle Schlauchverbindungen jeweils am distalen Ende aller Kupplungstücke 52 abgezogen. Der Überdruck im Blutgefäß 51 bleibt somit erhalten und die Innenräume des Gewebekultursystems steril. Nun können die Kappenteile 42, 43 und der Wärmemantel der Beschichtungseinrichtung entfernt werden. Nun kann das nach wie vor geschlossene zentrale Doppelrohrsystem (Rohre 3, 4) mit der Vene 51 entnommen, mit steriler PBS-Lösung an seinen Außenwänden abgespült und dann in den Operationssaal gesendet werden. Erst dort werden die Schraubendeckel 24, 25 entfernt. Dann wird das Blutgefäß 51 vorsichtig entnommen und sofort implantiert.

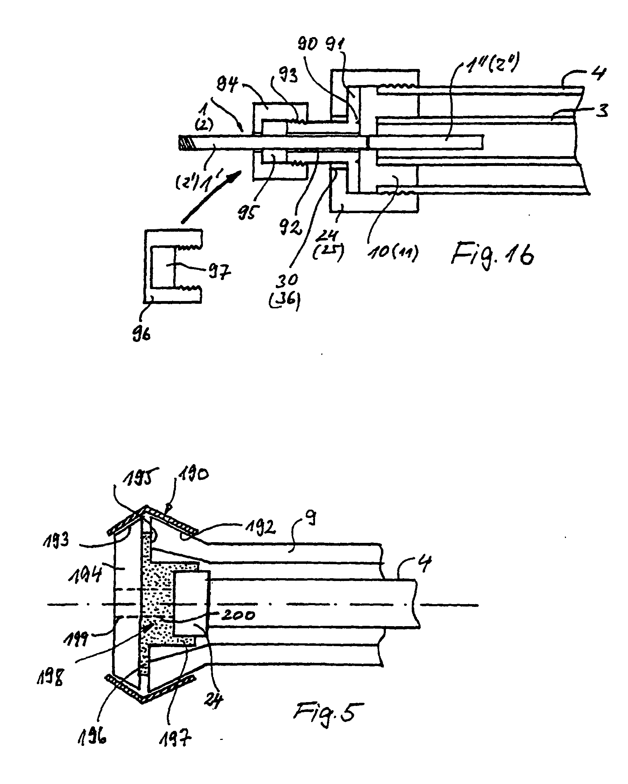

Die Figur 5 zeigt eine bevorzugte Weiterbildung der Erfindung, bei der der Deckel 42 der Figur 1a, der den Schraubdeckel 24 hält und zum Rohr 9 hin abdichtet, durch ein Kappenglied 198 ersetzt ist, das mit einem hohlzylindrischen Bereich 197 den Schraubdeckel 24 übergreift und mit einem Flansch 196 auf einem Auflageflansch 195 des Rohres 9 aufliegt. Der Flansch 196 liegt auch an einer kreisförmigen Platte 194, vorzugsweise einer Metallplatte, an, die außenseitig einen schrägen Rand 193 besitzt. Der Endbereich des Rohres 9 besitzt ebenfalls außenseitig einen schrägen Rand 192. Die schrägen Ränder 192, 193 bilden ein "V" oder dergleichen, das außenseitig durch einen komplementär ausgebildeten Spannring 190 übergriffen werden kann. Beim Spannen des an sich bekannten Spannringes 190 durch Betätigen eines Exzenterhebels werden die schrägen Ränder 192, 193 gegeneinander gezogen und werden dabei die Platte 194 und der Flansch 196 des Kappengliedes 198 gegen den Auflageflansch 195 des Rohres 9 zur Herstellung einer dichten Abdichtung gedrückt.

Vorzugsweise besteht das Kappenglied aus einem heiß polymerisierten Zwei-Komponenten Silikon-Kautschuk-Material.

In der Platte 194 und in dem Kappenglied 198 befinden sich vorzugsweise mittig Löcher 199, 2000, durch das die Endbereiche 7', 7'' des Schlauches 7, die Kanülen 1 bzw. 2 bzw. die Schlauchleitungen 34, 37 nach außen verlaufen können.

## Patentansprüche

1. Gewebekultursystem zur Epitheliasierung bzw. Endotheliasierung und zur funktionellen Untersuchung und Anlieferung natürlicher oder künstlicher Hohlorgane bzw. Gefäße unter kontrollierbaren Sterilbedingungen für chirurgische Implantationszwecke, **dadurch gekennzeichnet, daß** ein Hohlorgan (51) in einem ersten Rohr (3) anordenbar ist, wobei jeweils ein Ende des Hohlorganes (51) mit einer aus einer Seite des Rohres (3) dicht herausführbaren ersten oder zweiten Kanüle (1, 2) verbindbar ist, daß das erste Rohr (3) in einem zweiten Rohr (4) angeordnet ist, wobei zwischen dem ersten Rohr und dem zweiten Rohr eine an den Seiten des zweiten Rohres (4) abgedichtete Kammer (77) gebildet wird, daß das erste Rohr (3) Löcher (5) aufweist, die Verbindungen zwischen dem vom ersten Rohr (3) umschlossenen Raum (28) zu der Kammer (77) herstellen, daß um den Außenumfang des ersten Rohres (3) herum eine gasdurchlässige Leitung (7) verläuft, deren Endbereiche (7', 7'') dicht aus der Kammer (77) herausgeführt sind, daß durch eine Kanüle (1) in das Blutgefäß (51) ein patientenautologes Serum einfüllbar und während einer vorgegebenen ersten Zeitdauer inkubierbar ist und danach suspendierte Zellen des Patienten in vorgegebener Konzentration injizierbar sind, die eine zweite vorgegebene Zeitdauer im Blutgefäß (51) verbleiben, daß die Leitung (7) von einem Luftgemisch mit einem vorgegebenen Volumenanteil Kohlendioxid durchströmbar ist, daß in die Kammer (77) und über die Löcher (5) in den Raum (28) ein Kulturmedium über eine dicht von einer Seite des zweiten Rohres (4) her in die Kammer (77) eingeführte Leitung (34) einfüllbar ist und daß nach dem Entfernen der injizierten, suspendierten Zellen des Patienten aus dem Blutgefäß (51) ein Kulturmedium in das Blutgefäß (51) einbringbar ist, das während des Wachstums von an der Gefäßwand sedimentierten Zellen während einer dritten vorgegebenen Zeitdauer im Blutgefäß (51) unter hydrostatischem Überdruck verbleibt bzw. durch dieses perfundiert wird.

2. Gewebekultursystem nach Anspruch 1, **dadurch gekennzeichnet, daß** das erste Rohr (3) konzentrisch in dem zweiten Rohr (4) angeordnet ist.

3. Gewebekultursystem nach Anspruch 1 oder 2, **dadurch gekennzeichnet, daß** an jeder Seite des ersten Rohres (3) und des zweiten Rohres (4) eine erste bzw. zweite Quetschdichtung (10, 11) angeordnet ist, daß jeweils ein Endbereich des ersten Rohres (3) in eine erste Nut (14, 15) der ersten bzw. zweiten Quetschdichtung (10, 11) eingreift, daß jeweils ein Endbereich des zweiten Rohres (4) in eine zweite Nut (18, 19) der ersten bzw. zweiten Quetschdichtung (10, 11) eingreift, daß am Außenumfang des Endbereiches des zweiten Rohres (4) jeweils eine Befestigungseinrichtung für einen über der ersten bzw. zweiten Quetschdichtung (10, 11) anordenbaren Deckel (24, 25) vorgesehen ist, derart, daß bei befestigtem Deckel (24, 25) die erste bzw. zweite Quetschdichtung (10, 11) zusammengedrückt ist, und daß die erste bzw. zweite Kanüle (1, 2) dicht durch eine Bohrung (12, 13) der ersten bzw. zweiten Quetschdichtung (10, 11), die Endbereiche (7', 7'') der Leitung (7) dicht durch einen Durchgang (16, 17) einer Quetschdichtung (10) und die Leitungen (34, 37) dicht durch Durchgänge (35, 36) einer Quetschdichtung (11) verlaufen.

4. Gewebekultursystem nach Anspruch 3, **dadurch gekennzeichnet, daß** die Befestigungseinrichtung durch ein Außengewinde (20, 21) eines Endbereiches des zweiten Rohres (4) und ein Innengewinde (22, 23) des als Schraubdeckel (24, 25) ausgebildeten Deckels gebildet ist.

5. Gewebekultursystem nach einem der Ansprüche 1 bis 4, **dadurch gekennzeichnet, daß** die Löcher (4) in in Längsrichtung des ersten Rohres (3) verlaufenden Lochreihen angeordnet sind, die in Umfangsrichtung des ersten Rohres (3) voneinander beabstandet sind.

6. Gewebekultursystem nach einem der Ansprüche 3 bis 5, **dadurch gekennzeichnet, daß** die Endbereiche (7', 7'') der Leitung (7) und die erste Kanüle (1, 2) durch einen Durchgang (33) einer Deckelwand (26) eines Deckels (24) geführt sind.

7. Gewebekultursystem nach einem der Ansprüche 1 bis 7, **dadurch gekennzeichnet, daß** die zur Kammer (77) führenden Leitungen (34, 37) durch Durchgänge (35, 36) einer Quetschdichtung (11) geführt sind.

8. Gewebekultursystem nach Anspruch 7, **dadurch gekennzeichnet, daß** die zur Kammer (77) führenden Leitungen (34, 37) und die zweite Kanüle (2) durch einen Durchgang (32) einer Deckelwand (27) eines Deckels (25) verlaufen.

9. Gewebekultursystem nach einem der Ansprüche 1 bis 8, **dadurch gekennzeichnet, daß** die Enden der ersten Kanüle (1), der zweiten Kanüle (2), der Endbereiche (7', 7'') der Leitung (7) und der zur Kammer (77) führenden Leitungen (34, 37) jeweils mit einem Kupplungsstück (52) zur Verbindung mit externen Leitungen verbunden sind.

10. Gewebekultursystem nach einem der Ansprüche 1 bis 9, **dadurch gekennzeichnet, daß** das zweite Rohr (4) von einem Außenrohr (9) umgeben ist, das in der Nähe seiner Enden jeweils einen Zugangsstutzen (38, 39) aufweist, daß die Endbereiche des Außenrohres (9) zur Abdichtung des zwischen dem zweiten Rohr (4) und dem Außenrohr (9) gebildeten Raumes (67) dicht in eine Nut (44, 45) eines dicht auf den ersten bzw. zweiten Deckel (24, 25) aufgesetzten ersten bzw. zweiten Kappenteiles (42, 43) eingreifen und daß über die Zugangsstutzen (38, 39) in den Raum (67) eine Temperierflüssigkeit einbringbar ist.

11. Gewebekultursystem nach Anspruch 10, **dadurch gekennzeichnet, daß** das erste bzw. zweite Kappenteil (42, 43) auf dem jeweiligen Endbereich des Außenrohres (9) durch eine weitere Befestigungseinrichtung (40) gehalten wird.

12. Gewebekultursystem nach Anspruch 11, **dadurch gekennzeichnet, daß** die weitere Befestigungseinrichtung einen an dem jeweiligen Endbereich des Außenrohres (9) außenseitig angeordneten ersten bzw. zweiten Haltewulst (40, 41)umfaßt, der in den Innenumfang des ersten bzw. zweiten Kappenteiles (42, 43) dicht eingreift.

13. Gewebekultursystem nach einem der Ansprüche 1 bis 12, **dadurch gekennzeichnet, daß** die erste Quetschdichtung (10) und die zweite Quetschdichtung (11) und/oder der erste Deckel (24) und der zweite Deckel (25) und/oder das erste Kappenteil (42) und das zweite Kappenteil (43) und/oder die Kupplungsstücke (52) aus einem heiß polymerisierten Zwei-Komponenten Silikon-Kautschuk-Material bestehen.

14. Gewebekultursystem nach einem der Ansprüche 1 bis 13, **dadurch gekennzeichnet, daß** es eine Rotationseinrichtung (70) umfaßt, die das Außenrohr (9) und die damit verbundenen Elemente alternierend in die eine und in die andere Richtung dreht.

15. Gewebekultursystem nach Anspruch 14, **dadurch gekennzeichnet, daß** die Rotationseinrichtung (70) eine Antriebswalze (71) umfaßt, die am Außenumfang des Außenrohres (9) angreift.

16. Gewebekultursystem nach Anspruch 15, **dadurch gekennzeichnet, daß** das Außenrohr (9) mit seinem zwischen den Ausgangsstutzen (38, 39) angeordneten Bereich auf der Antriebswalze (71) und einer von dieser beabstandeten Walze (72) aufliegt.

17. Gewebekultursystem nach Anspruch 15 oder 16, **dadurch gekennzeichnet, daß** oberhalb des auf der Antriebswalze (71) und der Walze (72) aufliegenden Außenrohres (9) ein am Außenrohr (9) angreifendes Rad (73) vorgesehen ist, wobei das Rad (73) in einem verstellbaren Bügel (74) gehalten ist.

18. Gewebekultursystem nach einem der Ansprüche 15 bis 17, **dadurch gekennzeichnet, daß** die Länge der Antriebswalze (71) und der Walze (72) etwa dem Abstand der beiden Zugangsstutzen (38, 39) des Außenrohres (9) von einander entspricht.

19. Gewebekultursystem nach einem der Ansprüche 1 bis 18, **dadurch gekennzeichnet, daß** eine Temperiereinrichtung (80) vorgesehen ist, die zwei Gefäße (82, 83) aufweist, die jeweils eine Temperierflüssigkeit enthalten, wobei die Temperierflüssigkeiten über eine Wärmeaustauscheinrichtung (81) und den Raum (67) zwischen dem zweiten Rohr (4) und dem Außenrohr (9) in Reihe geschaltet sind, wobei die Luftvolumina der Gefäße (82, 83) oberhalb der Temperierflüssigkeiten durch eine Luftaustauschleitung (87) miteinander verbunden sind, und daß eine mechanische Vorrichtung vorgesehen ist, die die Gefäße (82, 83) abwechselnd gegenphasig zwischen alternierenden Niveaus (I, II) anhebt bzw. absenkt, so daß durch den Raum (67) zwischen dem zweiten Rohr (4) und dem Außenrohr (9) und die Wärmeaustauschereinrichtung (81) alternierend Temperierflüssigkeit zwischen den Gefäßen strömt.

20. Gewebekultursystem nach Anspruch 20, **dadurch gekennzeichnet, daß** die Wärmeaustauschereinrichtung (81) zusammen mit dem einen Gefäß (82) in einem auf einer vorgegebenen Temperatur gehaltenen Temperierbad angeordnet ist.

21. Gewebekultursystem nach Anspruch 20, **dadurch gekennzeichnet, daß** das Außenrohr (9) an seinen Enden jeweils einen Auflageflansch (195) aufweist, daß7) eines Kappengliedes (198) aufsetzbar ist, daß das Kappenglied (198) einen Flansch (196) besitzt, der auf dem Auflageflansch (195) des Außenrohres (9) aufliegt, daß eine Platte (194) auf der dem Deckelteil (24) abgewandten Seite anordenbar und auf den Flansch (196) aufsetzbar ist, und daß die Platte (194) und das Ende des Außenrohres (9) jeweils schräg nach außen verlaufen und eine Auswölbung, vorzugsweise ein "V" bilden, auf das ein komplementär ausgebildeter Spannring (190) aufsetzbar ist, der beim Spannen die Platte (198) gegen den Flansch (196) und diesen gegen den Auflageflansch (195) drückt.

22. Gewebekultursystem nach Anspruch 21, **dadurch gekennzeichnet, daß** das Kappenglied (198) aus einem heiß polymerisierten Zwei-Komponenten Silikon-Kautschuk-Material besteht.

23. Gewebekultursystem nach einem der Ansprüche 1 bis 22, **dadurch gekennzeichnet, daß** die Kanülen (1, 2) zweiteilig ausgebildet sind, und jeweils zwei miteinander verbindbare Kanülenteile (1', 1'', 2', 2'') umfassen, daß die Verbindungsstelle der Kanülenteile (1', 1'', 2', 2'') im Bereich der Quetschdichtungen (10, 11) positionierbar und zirkumferent abdichtbar sind, daß mit dem Deckelteil (24, 25) ein stempelartiges Teil (91) mit seiner Auflagefläche gegen die Quetschdichtung (10, 11) preßbar ist, wobei ein zylindrischer Bereich durch die Öffnung (30, 36) des Deckelteiles (24, 25) verläuft und daß das Kanülenteil (1', 2') durch eine Zentralbohrung (92) des zylindrischen Bereiches des stempelartigen Teiles (91) verläuft, und daß auf das freie Ende des zylindrischen Bereiches ein Deckel (94) mit einer Öffnung zum Hindurchführen des Kanülenteiles (1', 2') oder ein Abschlußdeckel (96) dicht aufsetzbar sind.

24. Verfahren zur Beschichtung eines Blutgefäßes (51) mit Zellen in einem Gewebekultursystem nach den Ansprüchen 1 bis 23, **gekennzeichnet durch** die folgende Schritte:
a) Dichtes Verbinden eines von ihrem nativen Endothel befreiten Blutgefäßes (51) eines Spenders an seinen Enden mit der ersten Kanüle (1) bzw. der zweiten Kanüle (2),
b) Anordnen des Blutgefäßes (51) und der damit verbundenen ersten Kanüle (1) und zweiten Kanüle (2) in dem ersten Rohr (3) derart, daß die zweite Kanüle (2) **durch** den Durchgang (13) der zweiten Quetschdichtung (11) verläuft, daß die erste Kanüle (1) und die Endbereiche (7', 7'') der Leitung (7) **durch** den Durchgang (12) bzw. die Durchgänge (16, 14) der ersten Quetschdichtung (10) verlaufen und daß die zur Kammer (77) führenden Durchgänge **durch** die zweite Quetschdichtung (11) verlaufen,
c) Befestigen des ersten Deckels (24) und des zweiten Dekkels (25) an dem zweiten Rohr (4),
d) Anordnen und Befestigen des Außenrohres (9) über dem zweiten Rohr (4) mit dem ersten Kappenteil (42) und dem zweiten Kappenteil (43) oder mit Kappengliedern (198), Platten (194) und Spannhebeln (190),
e) Durchströmen der Leitung (7) mit einem Luftgemisch mit einem vorbestimmten Volumenanteil Kohlendioxid,
f) Einbringen von patientenautologem Serum in das Blutgefäß (51) über eine Kanüle (1),
g) Alternierendes Durchströmen des Raumes zwischen dem zweiten Rohr (4) und dem Außenrohr (9) mit einer in der Temperiereinrichtung (80) temperierten Temperierflüssigkeit während einer ersten vorgegebenen Zeitdauer,
h) Entfernen des patientenautologen Serums aus dem Blutgefäß (51),
i) Füllen des Blutgefäßes (51) mit **durch** Kultivation vermehrten und anschließend suspendierten Epithelzellen des Patienten in einer genau bestimmten Konzentration,
j) Füllen der Kammer (77) mit einem Kulturmedium,
k) Alternierende Drehung des Außenrohres (9) in der Rotationseinrichtung (70) während einer zweiten vorgegebenen Zeitdauer, wobei die sedimentierten Endothelzellen an der Gefäßinnenwand anhaften,
l) Anhaltende Rotationseinrichtung (70) und Ausbringen des Inhaltes des Blutgefäßes (51) über eine Kanüle (2),
m) Füllen des Blutgefäßes (51) mit Kulturmedium und fortlaufende diskontinuierliche Perfusion des Blutgefäßes (51) mit dem Kulturmedium,
n) Entfernen des Außenrohres (9) und der Kappenteile (42, 43) oder der Kappenglieder (198), Platten (194) und Spannhebel (190) nach einer diskontinuierlichen Perfusion mit dem Kulturmedium während einer dritten vorgegebenen Zeitdauer und Abdichten der ersten Kanüle (1) und der zweiten Kanüle (2) und Transport des Blutgefäßes (51) zusammen mit dem ersten Rohr (3) und dem zweiten Rohr (4), die über die erste Quetschdichtung (10) und den ersten Deckel (24) sowie die zweite Quetschdichtung (11) und den zweiten Deckel (25) dicht miteinander verbunden sind, in einen Operationsraum und dort Entnahme des Blutgefäßes (51).

## Claims

1. A tissue culture system for epithelialisation or endothelialisation and for functionally testing and supplying natural or artificial hollow organs or vessels under controllable sterile conditions for the purpose of surgical implantations,
**characterised in that** a hollow organ (51) can be disposed in a first tube (3), where in each case one end of the hollow organ (51) can be connected to a first or second cannula (1, 2) which can be tightly passed out of one side of the tube (3),
**in that** the first tube (3) is disposed in a second tube (4), a chamber (77) sealed at the sides of the second tube (4) being formed between the first tube and the second tube,
**in that** the first tube (3) has holes (5) which produce connections between the space (28) enclosed by the first tube (3) to the chamber (77),
**in that** around the outer periphery of the first tube (3) there runs a gas-permeable line (7), the end regions (7', 7") of which are passed tightly out of the chamber (77),
**in that** a patient-autologous serum can be filled into the blood vessel (51) through a cannula (1) and can be incubated for a predetermined first period of time and after that suspended cells from the patient can be injected in a predetermined concentration, which remain in the blood vessel (51) for second predetermined period of time,
**in that** an air mixture having a predetermined volume percentage of carbon dioxide can flow through the line (7),
**in that** a culture medium can be filled into the chamber(77) and via the holes (5) into the space (28) via a line (34) which is introduced tightly from one side of the second tube (4) into the chamber (77),
**and in that** after the removal of the patient's injected, suspended cells from the blood vessel (51), a culture medium can be introduced into the blood vessel (51), which during the growth of cells which have sedimented on the vessel wall remains under hydrostatic positive pressure in the blood vessel (51) for a predetermined period of time or is perfused thereby.

2. A tissue culture system according to Claim 1,
**characterised in that** the first tube (3) is disposed concentrically in the second tube (4).

3. A tissue culture system according to Claim 1 or 2,
**characterised in that** a first or second pinching seal (10, 11) is provided at each side of the first tube (3) and of the second tube (4),
**in that** in each case an end region of the first tube (3) engages in a first groove (14, 15) of the first or second pinching seal (10, 11),
**in that** in each case an end region of the second tube (4) engages in a second groove (18, 19) of the first or second pinching seal (10, 11),
**in that,** on the outer periphery of the end region of the second tube (4), in each case a fastening device for a cover (24, 25) that can be arranged over the first or second pinching seal (10, 11) is provided in such a manner that, when the cover (24, 25) is fastened, the first or second pinching seal (10, 11) is compressed,
**and in that** the first or second cannula (1, 2) passes tightly through a bore (12, 13) of the first or second pinching seal (10, 11), the end regions (7', 7'') of the line (7) pass tightly through a passage (16, 17) of a pinching seal (10) and the lines (354, 37) pass tightly through passages (35, 36) of a pinching seal (11)

4. A tissue culture system according to Claim 3,
**characterised in that** the fastening device is formed by an external thread (20, 21) of an end region of the second tube (4) and an internal thread (22, 23) of the cover constructed as a screw lid (24, 25).

5. A tissue culture system according to one of Claims 1 to 4,
**characterised in that** the holes (4) are disposed in rows of holes which run in the longitudinal direction of the first tube (3) and are spaced from one another in the circumferential direction of the first tube (3).

6. A tissue culture system according to one of Claims 3 to 5,
**characterised in that** the end regions (7', 7'') of the line (7) and the first cannula (1, 2) are passed through a passage (33) in a cover wall (26) of a cover (24).

7. A tissue culture system according to one of Claims 1 to 7,
**characterised in that** the lines (34, 37) leading to the chamber (77) are passed through passages (35, 36) of a crushing seal (11).

8. A tissue culture system according to Claim 7,
**characterised in that** the lines (34, 37) leading to the chamber (77) and the second canula (2) run through a passage (32) in a cover wall (27) of a cover (25).

9. A tissue culture system according to one of Claims 1 to 8,
**characterised in that** the ends of the first canula (1), of the second canula (2), of the end regions (7', 7'') of the line (7) and of the lines (34, 37) leading to the chamber (77) are in each case connected to a coupling piece (52) for connection to external lines.

10. A tissue culture system according to one of Claims 1 to 9,
**characterised in that** the second tube (4) is surrounded by an outer tube (9), which in the vicinity of its ends in each case has an access connection piece (38, 39),
**in that** the end regions of the outer tube (9) for sealing the space (67) formed between the second tube (4) and the outer tube (9) engage tightly in a groove (44, 45) of a first or second cap part (42, 43) placed tightly onto the first or second cover (24, 25)
**and in that** a temperature control fluid can be introduced into the space (67) via the access connection pieces (38, 39).

11. A tissue culture system according to Claim 10,
**characterised in that** the first or second cap part (42, 43) is retained on the respective end region of the outer tube (9) by a further fastening device (40).

12. A tissue culture system according to Claim 11,
**characterised in that** the further fastening device comprises a first or second retaining bulb (40, 41), which is disposed on the outside at the respective end region of the outer tube (9) and tightly engages in the inner periphery of the first or second cap part (42, 43).

13. A tissue culture system according to one of Claims 1 to 12,
**characterised in that** the first pinching seal (10) and the second pinching seal (11) and/or the first cover (24) and the second cover (25) and/or the first cap part (42) and the second cap part (43) and/or the coupling pieces (52) are made from a hot-polymerised two-constituent silicon-rubber material.

14. A tissue culture system according to one of Claims 1 to 13,
**characterised in that** it comprises a rotation device (70) which alternately rotates the outer tube (9) and the elements connected thereto in the one and in the other direction.

15. A tissue culture system according to Claim 14,
**characterised in that** the rotation device (70) comprises a drive roller (71) which acts on the outer periphery of the outer tube (9).

16. A tissue culture system according to Claim 15,
**characterised in that** the outer tube (9) with its region disposed between the delivery connection piece (38, 39) lies on the drive roller (71) and a roller (72) spaced therefrom.

17. A tissue culture system according to Claim 15 or 16,
**characterised in that** a wheel (73) acting on the outer tube (9) is provided above the outer tube (9) that lies on the drive roller (71) and the roller (72), with the wheel (73) being held in an adjustable clamp (74).

18. A tissue culture system according to one of Claims 15 to 17,
**characterised in that** the length of the drive roller (71) and of the roller (72) corresponds roughly to the distance of the two access connection pieces (38, 39) of the outer tube (9) from each other.

19. A tissue culture system according to one of Claims 1 to 18,
**characterised in that** a temperature control device (80) is provided, which comprises two basins (82, 83), which in each case contain a temperature control fluid, with the temperature control fluids being connected in series via a heat exchanger device (81) and the space (67) between the second tube (4) and the outer tube (9), the volumes of air of the basins (82, 83) above the temperature control fluids being connected to each other by an air exchange line (87),
**and in that** a mechanical device is provided, which raises and lowers the basins (82, 83) alternately in anti-phase between alternating levels (I, II) so that temperature control fluid between the basins flows alternatively through the space (67) between the second tube (4) and the outer tube (9) and the heat exchanger device (81).

20. A tissue culture system according to Claim 20,
**characterised in that** the heat exchanger device (81) together with the one basin (82) is disposed in a temperature control bath kept at a predetermined temperature.

21. A tissue culture system according to Claim 20,
**characterised in that** the outer tube (9) comprises a bearing flange (195) at each of its ends,
**in that** a cap member (198) has a flange (196) which lies on the bearing flange (195) of the outer tube (9),
**in that** a plate (194) can be disposed on the side faced away from the cover part (23) and can be placed on the flange (196),
**and in that** the plate (194) and the end of the outer tube (9) in each case run diagonally outwards and form a bulge, preferably a V, onto which a complementary-formed clamping ring (190) can be placed, which, upon clamping, forces the plate (198) against the flange (198) and this against the bearing flange (195).

22. A tissue culture system according to Claim 21,
**characterised in that** the cap member (198) is made from a hot polymerised two-component silicon-rubber material.

23. A tissue culture system according to one of Claims 1 to 22,
**characterised in that** the cannulas (1, 2) are constructed in two parts, and in each case comprise two cannula parts that can be connected to each other (1', 1'', 2', 2''),
**in that** the connecting parts of the cannula parts (1', 1'', 2', 2'') can be positioned and circumferentially sealed in the region of the pinching seals (10, 11),
**in that** with the cover part (24, 25) a plunger-type part (91) can be pressed by its bearing face against the pinching seal (10, 11), with a cylindrical region passing through the opening (30, 36) in the cover part (24, 25)
**and in that** the cannula part (1', 2') passes through a central bore (92) of the cylindrical region of the plunger-type part (91),
**and in that** a cover (94) with an opening for the passage of the cannula part (1', 2') or a sealing cover (96) can be tightly placed onto the free end of the cylindrical region.

24. A method for coating a blood vessel (51) with cells in a cell culture system according to Claims 1 to 23,
**characterised by** the following steps:
a) tightly connecting a donor's blood vessel (51), which has been freed from its native endothelium, by its ends to the first cannula (1) or the second canula (2),
b) arranging the blood vessel (51) and the first cannula (1) and second cannula (2) connected therewith in the first tube (3) in such a manner that the second cannula (2) passes through the passage (13) of the second pinching seal (11), that the first cannula (1) and the end regions (7', 7'') of the line (7) pass through the passage (12) or the passages (16, 14) of the first pinching seal (10) and that the passages leading to the chamber (77) pass through the second pinching seal (11),
c) fixing the first cover (24) and the second cover (25) to the second tube (4),
d) arranging and fastening the outer tube (9) over/via the second tube (4) with the first cap part (42) and the second cap part (43) or with cap members (198), plates (194) and clamping levers (190),
e) passing an air mixture having a predetermined volume percentage of carbon dioxide through the line (7),
f) introducing patient-autologous serum into the blood vessel (51) via a cannula (1),
g) alternately flushing the space between the second tube (4) and the outer tube (9) with a temperature control fluid controlled in the temperature control device (80) for a first predetermined period,
h) removal of the patient-autologous serum from the blood vessel (51),
i) filling the blood vessel (51) with the patient's epithelial cells that have been reproduced by cultivation and have subsequently been suspended in a precisely determined concentration,
j) filing the chamber (77) with a culture medium,
k) alternating rotation of the outer tube (9) in the rotation device (70) for a second predetermined period, during which the sedimented endothelial cells adhere to the inner vessel wall,
l) constant rotation device (70) and discharge of the contents of the blood vessel (51) via a cannula (2),
m) filling the blood vessel (51) with culture medium and progressive discontinuous perfusion of the blood vessel (51) with the culture medium,
n) removal of the outer tube (9) and the cap parts (42, 43) or the cap members (198), plates (194) and clamping levers (190) after a discontinuous perfusion with the culture medium for a third predetermined period and sealing the first canula (1) and the second canula (2) and transport of the blood vessel (51) together with the first tube (3) and the second tube (4), which are connected tightly to each another via the first pinching seal (10) and the first cover (24) and also the second pinching seal (11) and the second cover (25), into an operating room and there removal of the blood vessel.

## Revendications

1. Système de culture cellulaire permettant l'épithélialisation ou l'endothélialisation et l'examen de la fonction d'organes creux ou de vaisseaux, naturels ou artificiels, et l'approvisionnement de ceux-ci, dans des conditions de stérilité contrôlées en vue d'une implantation chirurgicale, **caractérisé en ce que** l'on peut disposer un organe creux (51) dans un premier tube (3), chacune des extrémités de l'organe creux (51) pouvant être reliée respectivement à une première et une deuxième canule (1, 2) sortant de manière étanche d'un côté du tube (3), **en ce que** le premier tube (3) est disposé dans un deuxième tube (4), une chambre (77), fermée de manière étanche au niveau des côtés du deuxième tube (4) étant formée entre le premier tube et le deuxième tube, **en ce que** le premier tube (3) présente des trous (5) qui font communiquer l'espace (28) défini par le premier tube (3) et la chambre (77), **en ce que** le premier tube (3) est entouré sur sa circonférence d'une conduite (7) perméable aux gaz, dont les extrémités (7', 7") sortent de manière étanche de la chambre (77), qu'un sérum autologue du patient peut être introduit dans le vaisseau sanguin (51) au moyen d'une canule (1) et peut y être incubé pendant une première période déterminée, **en ce que** des cellules du patient en suspension peuvent ensuite y être injectées en une concentration déterminée, lesquelles cellules séjournent pendant une deuxième période déterminée dans le vaisseau sanguin, **en ce que** la conduite (7) peut être traversée par un mélange d'air contenant une fraction volumique de dioxyde de carbone prédéterminée, **en ce qu'**un milieu de culture peut être introduit dans la chambre (77) et, en passant par les trous (5), dans l'espace (28), au moyen d'une conduite (34) entrant de manière étanche dans la chambre (77) d'un côté du deuxième tube (4), et, **en ce qu'**après élimination des cellules injectées en suspension du vaisseau sanguin (51), un milieu de culture peut être introduit dans le vaisseau sanguin (51), lequel milieu de culture, pendant la croissance de cellules ayant sédimenté au niveau de la paroi du vaisseau, reste sous une suppression hydrostatique dans le vaisseau sanguin (51) pendant une troisième période d'une durée déterminée ou est perfusé à travers ledit vaisseau sanguin (51).

2. Système de culture cellulaire selon la revendication 1, **caractérisé en ce que** le premier tube est disposé de façon concentrique dans le deuxième tube (4).

3. Système de culture cellulaire selon la revendication 1 ou 2, **caractérisé en ce qu'**un premier et deuxième joints compressibles (10, 11) sont disposés de chaque côté du premier tube (3) et du deuxième tube (4), **en ce qu'**une partie d'extrémité du premier tube (3) s'engage dans une première gorge (14, 15) du premier ou deuxième joint compressible (10, 11), **en ce qu'**une partie terminale du deuxième tube (4) s'engage dans une deuxième gorge (18, 19) du premier ou deuxième joint compressible (10, 11), **en ce qu'**il est prévu sur la surface extérieure de la partie terminale du deuxième tube (4) un dispositif de fixation pour un couvercle (24, 25) pouvant être disposé sur le premier ou le deuxième joint compressible (10, 11), de telle sorte que lorsque le couvercle (24 ,25) est fixé, le premier ou deuxième joint compressible (10, 11) soit comprimé, et **en ce que** la première ou la deuxième canule (1, 2) s'étend de manière étanche à travers un trou (12, 13) dans le premier ou le deuxième joint compressible (10, 11), les parties terminales (7', 7") de la conduite (7) s'étendent de manière étanche à travers un passage (16, 17) d'un joint compressible (10), et les conduites (34, 37) s'étendent de manière étanche à travers des passages (35, 36) d'un joint compressible (11).

4. Système de culture cellulaire selon la revendication 3, **caractérisé en ce que** le dispositif de fixation et formé d'un filetage extérieur (20, 21) sur une partie terminale du deuxième tube (4) et un filetage intérieur (22, 23) du couvercle conformé en couvercle à visser (24, 25).

5. Système de culture cellulaire selon une des revendications 1 à 4, **caractérisé en ce que** les trous (4) sont disposés en rangées orientées dans la direction longitudinale du premier tube (3) qui sont mutuellement espacées dans la direction périphérique du premier tube (3).

6. Système de culture cellulaire selon une des revendications 3 à 5, **caractérisé en ce que** les parties terminales (7', 7") de la première conduite (7) et la première canule (1, 2) s'étendent à travers un passage (3) d'une paroi de couvercle (26) d'un couvercle (24).

7. Système de culture cellulaire selon une des revendications 1 à 6, **caractérisé en ce que** les conduites (34, 37) menant à la chambre (77) s'étendent à travers des passages (35, 36) d'un joint compressible (11).

8. Système de culture cellulaire selon la revendication 7, caractétisé en ce que les conduites (34, 37) menant à la chambre (77) et la deuxième canule (2) s'étendent à travers un passage (32) d'une paroi de couvercle (27) d'un couvercle (25).

9. Système de culture cellulaire selon une des revendications 1 à 8, **caractérisé en ce que** les extrémités de la première canule (1), de la deuxième canule (2), des parties terminales (7', 7") de la conduite (7) et des conduites (34, 37) menant à la chambre (77) sont munies chacune d'une pièce de couplage (52) pour la connexion avec des conduites externes.

10. Système de culture cellulaire selon une des revendications 1 à 9, **caractérisé en ce que** le deuxième tube (4) est entouré d'un tube extérieur (9) qui présente dans le voisinage de chacune de ses extrémités un raccord (38, 39), **en ce que** les parties terminales du tube extérieur (9), pour la fermeture étanche de l'espace (67) entre le deuxième tube (4) et le tube extérieur (9), s'engagent avec étanchéité dans une gorge (44, 45) d'un premier ou deuxième élément formant coiffe (42, 43) monté étanche sur le premier ou le deuxième couvercle (24, 25) et **en ce que** un liquide d'équilibrage de température peuve être chargé via les raccords (38, 39) dans la chambre (67).

11. Système de culture cellulaire scion la revendication 10, **caractérisé en ce que** le premier ou le deuxième élément formant coiffe (42, 43 est tenu par un dispositif de fixation (40) supplémentaire sur la partie terminale concernée du tube extérieur (9)

12. Système de culture cellulaire selon la revendication 11, **caractérisé en ce que** le dispositif de fixation supplémentaire comprend un premier on un deuxième renflement (40, 41) aménagé côté extérieur sur la partie terminale concernée du tube extérieur (9), qui s'engage de manière étanche dans le premier ou le deuxième élément formant coiffe (42, 43).

13. Système de culture cellulaire selon une des revendications 1 à 12 **caractérisé en ce que** le premier joint compressible (10) et le deuxième joint compressible (11) et/ou le premier couvercle (24) et le deuxième couvercle (25) et/ou le premier élément formant coiffe (42) et le deuxième élément formant coiffe (43) et/ou les pièces de couplage (52) sont réalisés en un caoutchouc siliconé à deux composants, polymérisé à chaud.

14. Système de culture cellulaire selon une des revendications 1 à 132, **caractérisé en ce qu'**il comprend un dispositif de rotation (70) qui fait tourner le tube extérieur (9)et les éléments liés à celui-ci alternativement dans l'une et dans l'autre direction.

15. Système de culture cellulaire selon la revendication 14, **caractérisé en ce que** le dispositif de rotation (70) comprend un cylindre d'entraînement (71) qui agit sur la surface extérieure du tube extérieur (9).

16. Système de culture cellulaire selon la revendication 15, **caractérisé en ce que** le tube extérieur (9) repose par sa partie située entre les raccords de sortie (38, 39) sur le cylindre d'entraînement (71) et sur un cylindre (72) écarté du premier.

17. Système de culture cellulaire selon la revendication 15 ou 16, **caractérisé en ce qu'**au dessus du tube extérieur (9) reposant sur le cylindre d'entraînement (71) et le cylindre (72) est prévue une roue (73) qui agit sur le tube extérieur (9), la roue (73) étant tenue dans un étrier (74) réglable.

18. Système de culture cellulaire selon une des revendications 15 à 17, **caractérisé en ce que** la longueur du cylindre d'entraînement (71) et du cylindre (72) correspond approximativement à la distance entre les deux raccords (38, 39) du tube extérieur (9).

19. Système de culture cellulaire selon une des revendications 1 à 18, **caractérisé en ce qu'**il est prévu un dispositif d'équilibrage de température (80) qui comprend deux récipients (82, 83) contenant chacun un liquide d'équilibrage de température, les liquides d'équilibrage de température étant connectés en série par l'intermédiaire d'un dispositif échangeur thermique (81) et de l'espace (67) entre le deuxième tube (4) et le tube extérieur (9), les volumes d'air des récipients (82, 83) au-dessus des liquide d'équilibrage de température étant reliés entre eux par une conduite d'échange d'air (87), et **en ce qu'**il est prévu un dispositif mécanique qui élève et abaisse alternativement, en opposition de phase, les récipients (82, 83) entre des niveaux alternés (I, II), de telle sorte du liquide d'équilibrage de température s'écoule alternativement entre les récipients dans l'espace (67) entre le deuxième tube (4) et le tube extérieur (9) et le dispositif échangeur thermique (81).

20. Système de culture cellulaire selon la revendication 20, **caractérisé en ce que** le dispositif échangeur thermique (81) avec l'un des récipients (82) est disposé dans un bain maintenu à une température prédéterminée.

21. Système de culture cellulaire selon la revendication 20, **caractérisé en ce que** le tube extérieur (9) comporte à chacune de ses extrémités un collet (195), **en ce que** l'élément coiffe (198) comporte un bord (196) qui repose sur le collet (195) du tube extérieur (9), **en ce qu'**une plaque (194) peut être disposée sur la face éloignée du couvercle (24) et sur le bord (196), et **en ce que** la plaque (194) et l'extrémité du tube extérieur (9) sont inclinées vers l'extérieur et forment un renflement, de préférence un « V », sur lequel peut être placé un collier de serrage (190) de forme complémentaire, qui lorsqu'il est serré, presse la plaque (198) contre le bord (196) et ce dernier contre le collet (195).

22. Système de culture cellulaire selon la revendication 21, **caractérisé en ce que** l'élément coiffe (198) est réalisé en caoutchouc siliconé à deux composants, polymérisé à chaud.

23. Système de culture cellulaire selon une des revendications 1 à 22, **caractérisé en ce que** les canules sont réalisées en deux parties et comprennent chaque fois deux parties de canule (1', 1", 2', 2") qui peuvent être liées l'une à l'autre, **en ce que** le point de jonction entre les parties de canule (1', 1", 2', 2") peut être positionné dans la région des joints compressibles (10, 11) et étanché sur son pourtour, **en ce qu'**avec le couvercle (24, 25) un élément presseur (91) peut être pressé contre le joint compressible (10, 11), une partie cylindrique s'étendant à travers l'ouverture (30, 36) du couvercle (24, 25), **en ce que** la partie de canule (1', 2') s'étend à travers un trou central (92) de la partie cylindrique de l'élément presseur (91) et **en ce qu'**un couvercle (94) pourvu d'un orifice pour le passage de la partie de canule (1', 2') ou un obturateur (96) peut êtré monté étanche sur l'extrémité libre de la partie cylindrique.

24. Procédé de revêtement d'un vaisseau sanguin (51) avec des cellules dans un système de culture cellulaire selon les revendications 1 à 28, **caractérisé par** les étapes suivantes :
a) raccordement étanche d'un vaisseau sanguin (51) d'un donneur, débarrassé de son endothélium natif, au niveau de ses extrémités avec la première canule (1) et la deuxième canule (2),
b) placement du vaisseau sanguin (51) avec la première canule (1) et la deuxième canule (2) liées à celui-ci dans le premier tube (3), de telle sorte que la deuxième canule (2) s'étende à travers le passage (13) dans le deuxième joint compressible (11), que la première canule (1) et les parties d'extrémité (7', 7'') de la conduite (7) s'étendent à travers le passage (12) ou les passages (16, 14) du premier joint compressible (10) et que les passages menant à la chambre (77) s'étendent à travers le deuxième joint compressible (11),
c) fixation du premier couvercle (24) et du deuxième couvercle (25) sur le deuxième tube (4)
d) disposition et fixation du tube extérieur (9) sur le deuxième tube (4) avec le premier élément formant coiffe (42) et le deuxième élément formant coiffe (43) ou avec des éléments coiffe (198), des plaques (194) et des leviers de serrage (190),
e) circulation dans la conduite (7) d'un mélange d'air avec une fraction volumique prédéterminée de dioxyde de carbone,
f) introduction de sérum autologue du patient dans le vaisseau sanguin (51) à l'aide d'un canule (1),
g) circulation alternée dans l'espace entre le deuxième tube (4) et le tube extérieur (9) d'un liquide d'équilibrage de température tempéré dans un dispositif d'équilibrage de température (80) pendant une première période prédéterminée,
h) élimination du sérum autologue du vaisseau sanguin,
i) remplissage du vaisseau sanguin (51) avec des cellules d'épithélium du patient multipliées par culture et mises en suspension en une concentration très précise,
j) remplissage de la chambre (77) avec un milieu de culture,
k) rotation alternée du tube extérieur (9) dans le dispositif de rotation (70), pendant une deuxième période prédéterminée, les cellules d'endothélium sédimentées adhérant à la paroi intérieure du vaisseau,
l) arrêt du dispositif de rotation (70) et extraction du contenu du vaisseau sanguin (51) à l'aide d'une canule (2),
m) remplissage du vaisseau sanguin (51) avec le milieu de culture et perfusion discontinue du vaisseau sanguin (51) avec le milieu de culture,
n) retrait du tube extérieur (9) et des éléments formant coiffe (42, 43) ou des éléments coiffe (198), plaques (194) et leviers de serrage (190), après une perfusion discontinue avec le milieu de culture pendant une troisième période prédéterminée, fermeture étanche de la première canule (1) et de la deuxième canule (2) et acheminement du vaisseau sanguin (51) avec le premier tube (3) et le deuxième tube (4) reliés entre eux de manière étanche par le premier joint compressible (10) et le premier couvercle (24) ainsi que par le deuxième joint compressible (11) et le deuxième couvercle (25), dans une salle d'opération et là extraction du vaisseau sanguin (51).
